# EUROPEAN PATENT APPLICATION

(11) **EP 4 817 625 A1**
(43) Date of publication of application: **07.10.2026**
(21) Application number: 24898148.2
(22) Date of filing: 28.11.2024
(51) Int. Cl.: A61K 38/21, A61K 47/68, A61P 35/00, A61P 31/00, C07K 14/555

(54) **NOVEL USE OF INTERFERON EPSILON**

(30) Priority: 28.11.2023 KR 20230168553; 27.11.2024 KR 20240172093
(71) Applicant: Abion Inc., Seoul 08394 (KR); Seoul National University R & DB Foundation, Seoul 08826 (KR)
(72) Inventor: SHIN, Young Kee, Seoul 08826 (KR); PARK, Hee Geon, Seoul 08394 (KR); CHOI, Jun Young, Seoul 08394 (KR); KIM, Na Young, Seoul 08394 (KR); LEE, Sae Hyung, Seoul 08394 (KR)
(74) Representative: Schwarz & Partner Patentanwälte GmbH
(86) International application number: PCT/KR2024/019100
(87) International publication number: WO 2025/116554

(57) **Abstract**

The present invention relates to novel use of interferon epsilon and, more specifically, to a pharmaceutical composition for the prevention or treatment of diseases, comprising interferon epsilon as an active ingredient. The pharmaceutical composition has significantly improved activity in a slightly acidic environment compared to a neutral environment, and thus can be administered systemically without separate treatment such as targeting or masking for controlling systemic toxicity, and therefore, can be effectively used for the treatment of related diseases.

## Description

### [Technical Field]

The present application claims priority to Korean Patent Application No. 10-2023-0168553 filed on November 28, 2023, and Korean Patent Application No. 10-2024-0172093 filed on November 27, 2024, the entire disclosures of which are incorporated herein by reference.

The present invention relates to a novel use of interferon epsilon, and more specifically, to a pharmaceutical composition for preventing or treating a disease comprising interferon epsilon as an active ingredient, wherein the interferon epsilon is activated in tissue with decreased pH due to the pathological state of the disease, a fusion protein comprising an antibody or a fragment thereof directly or indirectly bound to the interferon epsilon, and a pharmaceutical composition comprising the fusion protein.

### [Background Art]

Interferon (IFN) is a protein belonging to cytokines, which are molecules known to be used for intercellular communication to trigger the immune system in the body. Interferon was named for its ability to "interfere" with the replication of viruses by protecting cells from viral infections. Additionally, it has functions such as activating immune cells like natural killer cells and macrophages, increasing the expression of major histocompatibility complex (MHC) antigens to upregulate antigen presentation, thereby enhancing the host's defense mechanisms.

Interferons are classified into Type I, which includes IFN-α, IFN-β, IFN-ε, IFN-κ, and IFN-ω; Type II, which includes IFN-γ; and Type III, which includes IFN-λ. Among these, particularly Type I interferons, especially IFN-α and IFN-β, are widely used as therapeutics for various diseases. For example, IFN-α2a, IFN-α2b, and pegylated IFN-α2b are used as treatments for diseases such as blast cell leukemia, melanoma, renal cell carcinoma, Kaposi's sarcoma, multiple myeloma, follicular and non-Hodgkin's lymphoma, and chronic myelogenous leukemia. Human IFN-β has also been approved in Japan as a treatment for gliomas, medulloblastomas, astrocytomas, and melanomas. Additionally, IFN-β1 has been approved by the FDA for use as a treatment for multiple sclerosis.

As such, interferons have demonstrated efficacy in numerous cancers. However, side effects such as fever, chills, myalgia, headache, nausea, and fatigue, which are similar to flu-like symptoms, may occur. In particular, the anti-tumor effects of systemic interferon therapy are often accompanied by severe side effects, including inflammation and direct toxicity to tissues. Furthermore, due to the short half-life of interferons, the dosing interval inevitably becomes shorter, which ultimately results in an increase in side effects.

Methods to control systemic toxicity caused by interferons include delivering interferons directly to tumor sites using the tumor-targeting ability of monoclonal antibodies, conjugating polyethylene glycol (PEG) to interferons to increase their half-life, and suppressing (masking) the activity of interferons until they reach the tumor. These methods are known in the art. However, tumor-targeted interferons can still be recognized by interferon receptors expressed throughout the body, leading to side effects. There is also the issue that the side effects of PEGylated interferons are not substantially different from those of non-PEGylated interferons. Additionally, masked interferons have drawbacks such as complex manufacturing processes, variability in interferon activity depending on the activity of proteases in the tumor microenvironment, and the potential for additional protein structures used for masking to induce new immune responses.

Therefore, there is a need for a novel strategy that can suppress systemic toxicity caused by systemic administration of interferons while specifically inducing interferon activity in pathological tissues.

### [Summary of Invention]

### [Problem to be Solved]

The present inventors have newly identified that interferon epsilon (IFN-ε) exhibits a characteristic of a sharp increase in activity in an environment with low pH. Due to this characteristic, it was confirmed that even when interferon epsilon was systemically administered to a tumor mouse model, the nonspecific immune activity in the blood and spleen was lower compared to the interferon beta (IFN-β) administration group, while the degree of immune activity within the tumor was similar, thereby completing the present invention.

Accordingly, an object of the present invention is to provide a pharmaceutical composition for preventing or treating a disease comprising interferon epsilon as an active ingredient, wherein the interferon epsilon is activated in tissue with decreased pH due to the pathological state of the disease.

Another object of the present invention is to provide a fusion protein comprising the interferon epsilon and an antibody or a fragment thereof directly or indirectly bound to the interferon epsilon.

Yet another object of the present invention is to provide a pharmaceutical composition for preventing or treating a disease comprising the fusion protein, wherein the fusion protein is activated in tissue with decreased pH due to the pathological state of the disease.

Still another object of the present invention is to provide a pharmaceutical composition consisting of interferon epsilon for preventing or treating a disease.

A further object of the present invention is to provide a pharmaceutical composition consisting essentially of interferon epsilon for preventing or treating a disease.

### [Means for Solving the Problem]

To achieve the above objectives, the present invention provides a pharmaceutical composition for preventing or treating a disease comprising interferon epsilon as an active ingredient, characterized in that the interferon epsilon is activated in tissue with decreased pH due to a pathological state of the disease.

To achieve another objective of the present invention, the present invention provides a fusion protein comprising the interferon epsilon and an antibody or a fragment thereof directly or indirectly bound to the interferon epsilon.

To achieve yet another objective of the present invention, the present invention provides a pharmaceutical composition for preventing or treating a disease comprising the fusion protein, wherein the fusion protein is activated in tissue with decreased pH due to a pathological state of the disease.

To achieve yet another objective of the present invention, the present invention provides a pharmaceutical composition consisting of interferon epsilon for preventing or treating a disease.

To achieve yet another objective of the present invention, the present invention provides a pharmaceutical composition consisting essentially of interferon epsilon for preventing or treating a disease.

Hereinafter, the present invention will be described in detail.

Meanwhile, each description and embodiment disclosed in the present invention can be applied to other descriptions and embodiments. That is, all combinations of various elements disclosed in the present invention fall within the scope of the present invention. Furthermore, the scope of the present invention cannot be considered limited by the specific descriptions provided below.

In this specification, the term "comprising" is used in the same sense as "including" or "characterized by" and does not exclude additional components not specifically mentioned in the composition according to the present invention. Additionally, the term "consisting of" means excluding additional elements or components not separately described. The term "consisting essentially of" means that the composition may include substances that do not substantially affect its fundamental characteristics, in addition to the described substances.

The term "treatment" as used in the present invention comprehensively refers to improving symptoms caused by a disease, including temporarily or permanently alleviating symptoms, eliminating the cause of symptoms, or preventing or delaying the manifestation of symptoms of a disease or pathological condition. This may include curing the disease, substantially preventing it, or improving the condition, as well as alleviating, curing, or preventing one or most symptoms caused by the disease, but is not limited thereto.

The term "prevention" as used in the present invention refers to any act of suppressing symptoms or delaying the onset of a disease through the administration of the pharmaceutical composition according to the present invention.

The term "improvement" as used in the present invention refers to any act of reducing parameters associated with an abnormal condition, such as the severity of symptoms.

The terms or words used in the description and claims of the present invention should not be interpreted as being limited to their conventional or dictionary meanings. Instead, they should be interpreted based on the principle that the inventor can appropriately define the concepts of terms to best explain their invention, and they should be understood in a manner consistent with the technical spirit of the present invention.

The present invention provides a pharmaceutical composition for preventing or treating a disease comprising interferon epsilon as an active ingredient, characterized in that the interferon epsilon is activated in tissue with decreased pH due to a pathological state of the disease.

In the present invention, the interferon epsilon (interferon epsilon, IFN-ε) refers to a cytokine belonging to type I interferons mediated through binding to interferon alpha/beta receptor (IFNAR1 and IFNAR2), which activates the JAK-STAT pathway through its interaction with the receptor. The interferon epsilon exists in various organisms, including humans, mice, pigs, cattle, dogs, and horses, and is known to be primarily expressed in various mucosal tissues such as the lungs, small intestine, and reproductive organs, unlike interferon alpha (hereinafter, IFN-α) and beta (hereinafter, IFN-β). Meanwhile, in the present invention, the interferon epsilon preferably refers to human interferon epsilon but is not limited thereto. Furthermore, in this specification, the interferon epsilon may be referred to as interferon epsilon, interferon-ε, interferon-e, IFN-ε, IFN-e, IFNε, or IFNe, all of which refer to the same interferon epsilon.

According to one embodiment of the present invention, IFN-α and IFN-ω showed decreased activity under mildly acidic conditions (pH 6.0) compared to neutral conditions (pH 7.4), while IFN-β and IFN-ε exhibited increased activity under mildly acidic conditions, with IFN-ε showing a particularly significant difference. More specifically, IFN-ε demonstrated a 5.02-fold improvement in mildly acidic environments compared to neutral environments, and even when treated with 43.76 times lower receptor concentrations in mildly acidic environments, it exhibited superior binding ability compared to neutral environments. Furthermore, it was confirmed that pSTAT-1 signaling was activated even at concentrations more than 1000 times lower in mildly acidic environments compared to neutral environments. Therefore, the interferon epsilon according to the present invention is characterized by its activity being exhibited in tissues with a pH below 7, i.e., tissues with decreased pH compared to normal tissues. The pH of the "tissue with decreased pH" is preferably pH 5.0 to pH 6.9, pH 5.0 to pH 6.8, pH 5.0 to pH 6.7, pH 5.0 to pH 6.6, pH 5.0 to pH 6.5, pH 5.0 to pH 6.4, pH 5.0 to pH 6.3, pH 5.0 to pH 6.2, pH 5.0 to pH 6.1, pH 5.0 to pH 6.0, pH 5.0 to pH 5.9, pH 5.0 to pH 5.8, pH 5.0 to pH 5.7, pH 5.0 to pH 5.6, pH 5.0 to pH 5.5, pH 5.0 to pH 5.4, pH 5.0 to pH 5.3, pH 5.0 to pH 5.2, or pH 5.0 to pH 5.1, but is not limited thereto. Additionally, as described above, the interferon epsilon exhibits increased activity in tissues with decreased pH compared to normal tissues, characterized by a 2-fold, 3-fold, 4-fold, 5-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 1500-fold, 2000-fold, 3000-fold, 4000-fold, 5000-fold, 10000-fold, or more increase. Preferably, the activity increases by 2-fold to 10-fold, 2-fold to 50-fold, 2-fold to 100-fold, 2-fold to 200-fold, 2-fold to 300-fold, 2-fold to 400-fold, 2-fold to 500-fold, 2-fold to 600-fold, 2-fold to 700-fold, 2-fold to 800-fold, 2-fold to 900-fold, 2-fold to 1000-fold, 2-fold to 1500-fold, 2-fold to 2000-fold, 2-fold to 3000-fold, 2-fold to 4000-fold, 2-fold to 5000-fold, 2-fold to 10000-fold, or more; or 3-fold to 10-fold, 3-fold to 50-fold, 3-fold to 100-fold, 3-fold to 200-fold, 3-fold to 300-fold, 3-fold to 400-fold, 3-fold to 500-fold, 3-fold to 600-fold, 3-fold to 700-fold, 3-fold to 800-fold, 3-fold to 900-fold, 3-fold to 1000-fold, 3-fold to 1500-fold, 3-fold to 2000-fold, 3-fold to 3000-fold, 3-fold to 4000-fold, 3-fold to 5000-fold, 3-fold to 10000-fold, or more; or 4-fold to 10-fold, 4-fold to 50-fold, 4-fold to 100-fold, 4-fold to 200-fold, 4-fold to 300-fold, 4-fold to 400-fold, 4-fold to 500-fold, 4-fold to 600-fold, 4-fold to 700-fold, 4-fold to 800-fold, 4-fold to 900-fold, 4-fold to 1000-fold, 4-fold to 1500-fold, 4-fold to 2000-fold, 4-fold to 3000-fold, 4-fold to 4000-fold, 4-fold to 5000-fold, 4-fold to 10000-fold, or more; or 5-fold to 10-fold, 5-fold to 50-fold, 5-fold to 100-fold, 5-fold to 200-fold, 5-fold to 300-fold, 5-fold to 400-fold, 5-fold to 500-fold, 5-fold to 600-fold, 5-fold to 700-fold, 5-fold to 800-fold, 5-fold to 900-fold, 5-fold to 1000-fold, 5-fold to 1500-fold, 5-fold to 2000-fold, 5-fold to 3000-fold, 5-fold to 4000-fold, 5-fold to 5000-fold, 5-fold to 10000-fold, or more, but is not limited thereto.

Accordingly, the interferon epsilon according to the present invention is characterized by being capable of systemic administration without special processing such as targeting or masking. Particularly, the pH of the tumor microenvironment is known to be approximately pH 5.6 to 6.8, and when using the interferon epsilon according to the present invention for treating cancer and/or tumors, there is an advantage of reducing side effects even when systemic administration therapy is employed. In fact, according to one embodiment of the present invention, when interferon beta and epsilon were intraperitoneally administered to a tumor-induced mouse model using the B16F10 cell line constituting a low pH tumor microenvironment, the interferon epsilon administration group showed less reduction in white blood cell count and myeloid cells in the blood compared to the interferon beta administration group, and excessive pSTAT-1 activation did not occur. On the other hand, the level of T cell infiltration within the tumor and the reduction of myeloid cells were similar to those of the interferon beta administration group.

Meanwhile, in the present invention, the "systemic administration" refers to administering the composition according to the present invention into the circulatory system to affect the entire body, and preferably includes intravenous, intramuscular, intraarterial, intramedullary, intrathecal, intracardiac, transdermal, subcutaneous, intraperitoneal, intranasal, enteral, topical, sublingual, intrarectal, or intratumoral administration, but is not limited thereto.

The composition comprising interferon epsilon as an active ingredient according to the present invention can be applied without limitation to diseases and/or pathological conditions that can be treated using the currently known interferon epsilon.

Generally, STAT-1 signaling induced by IFNAR1/IFNAR2 activation by interferons is known to promote the exposure of MHC class I molecules on the surface of cancer cells, and in combination with the upregulation of tumor-associated antigens, it is known in the art to overall increase the antigenicity of tumors. Therefore, the pathological condition may refer to cancer and/or tumors.

In the present invention, the cancer and/or tumor is preferably at least one selected from ovarian cancer, papilloma, laryngeal papilloma, human papilloma, bladder cancer, and cervical cancer, but the cancer and/or tumor that can be treated with the composition according to the present invention is not particularly limited and includes both solid tumors and hematologic cancers. Non-limiting examples of the cancer and/or tumor include hairy cell leukemia, chronic myelogenous leukemia, multiple myeloma, non-Hodgkin's lymphoma, skin cancers such as melanoma, Kaposi's sarcoma, kidney cancer, lung cancer, brain tumors such as glioma, breast cancer, colorectal cancer, liver cancer, hepatocellular carcinoma, gastric cancer, bronchial cancer, nasopharyngeal cancer, laryngeal cancer, pancreatic cancer, colon cancer, pancreatic cancer, prostate cancer, bone cancer, thyroid cancer, parathyroid cancer, esophageal cancer, bile duct cancer, testicular cancer, rectal cancer, head and neck cancer, cervical cancer, ureteral cancer, neuroblastoma, fibrosarcoma, rhabdomyosarcoma, astrocytoma, glioblastoma, neuroblastoma, and malignant mesothelioma, but are not limited thereto.

Additionally, interferons activate interferon-stimulated genes (ISGs) through the JAK-STAT pathway, and many ISGs are known in the art to directly target pathways and functions necessary during the life cycle of pathogens, thereby controlling viral, bacterial, and parasitic infections. Therefore, the pathological condition may refer to infectious diseases.

In the present invention, the infectious disease may preferably refer to viral infections. Non-limiting examples of the viral infections include AIDS caused by HIV, hepatitis B and C virus infections, but are not limited thereto, and may further include one or more selected from the group consisting of influenza virus infections, respiratory syncytial virus infections, herpes virus infections, human papilloma virus infections, coronavirus infections, and other viral infections, but are not limited thereto.

The composition according to the present invention comprises the aforementioned interferon epsilon as an active ingredient and can be formulated in an appropriate form together with pharmaceutically acceptable carriers, and may further contain excipients or diluents. The term "pharmaceutically acceptable" refers to a non-toxic composition that is physiologically acceptable and does not cause allergic reactions such as gastrointestinal disturbances, dizziness, or similar reactions when administered to humans. The pharmaceutically acceptable carriers may include, for example, carriers for oral administration or carriers for parenteral administration. Carriers for oral administration may include lactose, starch, cellulose derivatives, magnesium stearate, stearic acid, and the like. Additionally, carriers for parenteral administration may include water, suitable oils, saline, aqueous glucose, and glycols, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. The pharmaceutical composition of the present invention may further include lubricants, wetting agents, sweeteners, flavoring agents, emulsifiers, suspending agents, and the like. Other pharmaceutically acceptable carriers and formulations can be referenced in the following literature.

As described above, the composition according to the present invention can be administered systemically and can be administered to mammals, including humans, by any method, for example, orally or parenterally. Therefore, the composition according to the present invention can be formulated as a preparation for oral administration or parenteral administration according to the administration routes described above.

In the case of preparations for oral administration, the composition of the present invention can be formulated into powders, granules, tablets, pills, sugar-coated tablets, capsules, liquids, gels, syrups, slurries, suspensions, and the like using methods known in the art. For example, oral preparations can be obtained by mixing the active ingredient with solid excipients, grinding the mixture, adding suitable auxiliaries, and processing it into a granulated mixture to produce tablets or sugar-coated tablets. Examples of suitable excipients include sugars such as lactose, dextrose, sucrose, sorbitol, mannitol, xylitol, erythritol, and maltitol; starches such as corn starch, wheat starch, rice starch, and potato starch; celluloses such as cellulose, methylcellulose, sodium carboxymethylcellulose, and hydroxypropylmethylcellulose; and fillers such as gelatin and polyvinylpyrrolidone. Additionally, disintegrants such as cross-linked polyvinylpyrrolidone, agar, alginic acid, or sodium alginate may be added as needed. Furthermore, the pharmaceutical composition of the present invention may further include anti-caking agents, lubricants, wetting agents, flavoring agents, emulsifiers, and preservatives.

In the case of preparations for parenteral administration, the composition can be formulated into injections, creams, lotions, topical ointments, oils, moisturizers, gels, aerosols, and nasal inhalants using methods known in the art.

The total effective amount of the composition according to the present invention can be administered to a patient as a single dose or as a multiple dose over a long period through a fractionated treatment protocol. The composition according to the present invention may vary the content of the active ingredient depending on the severity of the disease. Preferably, the total preferred dosage of the composition according to the present invention may be about 0.01 µg to 10,000 mg per kg of body weight of the patient or subject per day, and most preferably 0.1 µg to 500 mg. However, the dosage of the composition is determined by considering various factors such as the formulation method, administration route, frequency of treatment, as well as the patient's age, weight, health condition, gender, severity of the disease, diet, and excretion rate. Therefore, those skilled in the art will be able to determine the appropriate effective dosage of the composition of the present invention by taking these factors into account. The composition according to the present invention is not particularly limited in its formulation, administration route, or administration method as long as it exhibits the effects of the present invention.

The term "effective amount" in the present invention refers to an amount that, when administered to a subject, exhibits effects such as improvement, treatment, detection, diagnosis, inhibition, or reduction of cancer or infectious diseases. The term "subject" may refer to an animal, preferably a mammal, particularly a human, and may also include cells, tissues, organs, etc., derived from animals. The subject may be a patient in need of the aforementioned effects.

In the present invention, the content of the composition is not significantly limited depending on the purpose or aspect of use, and, for example, it may be 0.01 to 99% by weight, preferably 0.5 to 50% by weight, and more preferably 1 to 30% by weight, based on the total weight of the composition. In addition, the pharmaceutical composition according to the present invention may further comprise additives such as pharmaceutically acceptable carriers, excipients, or diluents in addition to the active ingredient. The pharmaceutical composition of the present invention may comprise interferon epsilon prepared by the method of the present invention in an amount of 0.1 to 99.9% by weight and a carrier in an amount of 99.9% to 0.1% by weight.

In addition, the present invention provides a fusion protein comprising the above-described interferon epsilon and an antibody or a fragment thereof directly or indirectly bound to the interferon epsilon.

In the fusion protein according to the present invention, the meaning of the interferon epsilon is the same as described in the above-described composition, and hereinafter, the specific configuration of the fusion protein will be described.

In the present invention, the antibody includes monoclonal antibodies, polyclonal antibodies, multispecific antibodies (e.g., bispecific antibodies), and fragments thereof. A complete antibody generally has a Y-shaped structure and consists of two long heavy chains (H) and two short light chains (L). Each heavy chain and light chain are connected to each other by disulfide bonds and are divided into a variable region (V), which is the site that reacts with antigens, and a constant region (C), which is the site that exhibits effector functions. The variable region contains complementarity-determining regions (CDRs) that determine the structure of the variable region to form specific binding with antigens and regulate antibody binding strength. The fragment of the antibody refers to a specific part that can react with an antigen and exhibit antigen-binding activity, and examples include Fab fragments (fragments by papain digestion), Fab' fragments (fragments by pepsin digestion and partial reduction), F(ab')2 fragments (fragments by pepsin digestion), Facb (fragments by plasmin digestion), Fd (fragments by pepsin digestion, partial reduction, and reaggregation), scFv fragments (fragments by molecular biology techniques), and the like. Preferably, the antibody is an antibody or a fragment thereof that recognizes tumor-specific antigens and can be used as a targeted therapeutic agent for diseases such as cancer. Examples include trastuzumab, an antibody therapeutic agent targeting human epidermal growth factor receptor (HER-2); cetuximab, an antibody therapeutic agent targeting epidermal growth factor receptor (EGFR); atezolizumab, an antibody therapeutic agent targeting PD-L1 expressed on the surface of cancer cells; and sacituzumab, an antibody therapeutic agent targeting TROP2 expressed on the surface of cancer cells.

The fusion protein according to the present invention may be a fusion protein formed by linking interferon epsilon and an antibody or a fragment thereof via a linker, preferably a peptide linker. The linker refers to a molecule that connects two or more distinct substances to each other, consisting of short fragments of amino acids or amino acid analogs connected by peptide bonds. In this case, glycine, serine, alanine, and the like may be used as the main constituent amino acids, and glycine-serine linkers, glycine-serine-alanine linkers, and the like may be used. Such linkers may be connected to the C-terminus of the heavy chain of the antibody or the C-terminus of the light chain of the antibody, or the N-terminus of the linker may be connected to both the C-terminus of the light chain and the C-terminus of the heavy chain of the antibody. At this time, the N-terminus of the interferon epsilon may be connected to the C-terminus of the linker.

In the fusion protein according to the present invention, since the antibody recognizes tumor-specific antigens and is intended for targeted therapy for diseases such as cancer, it is evident that the pharmacological effect of the fusion protein will be substantially the same as that of the above-described interferon epsilon. Accordingly, the present invention provides a composition comprising the fusion protein for tissue-specific treatment or prevention, wherein the tissue has decreased pH due to a pathological condition.

In the composition according to the present invention, the meanings of all terms, including the fusion protein, pathological condition, pH, and tissue with decreased pH, are the same as described in the above-described composition, and the contents described in the relevant sections can be directly applied to the composition comprising the fusion protein according to the present invention.

### [Effect of the Invention]

The interferon epsilon according to the present invention is characterized by significantly enhanced activity in a mildly acidic environment compared to a neutral environment. Therefore, it has the advantage of being capable of systemic administration without requiring additional processes such as targeting or masking to control systemic toxicity, and thus can be usefully utilized in the treatment of related diseases.

### [Brief Description of the Drawings]

Fig. 1a to Fig. 1f illustrate the results of evaluating receptor binding ability in neutral and acidic environments for each type of type I interferon (IFN-α2b, IFN-β, IFN-β R27T, IFN-β C17S/R27T, IFN-ω, and IFN-ε).
Fig. 2a and Fig. 2b illustrate the receptor binding ability in neutral and acidic environments for each type of type I interferon, standardized to neutral conditions.
Fig. 3a and Fig. 3b illustrate the changes in binding ability of interferon epsilon to receptors IFNAR1 and IFNAR2 under acidic conditions compared to neutral conditions.

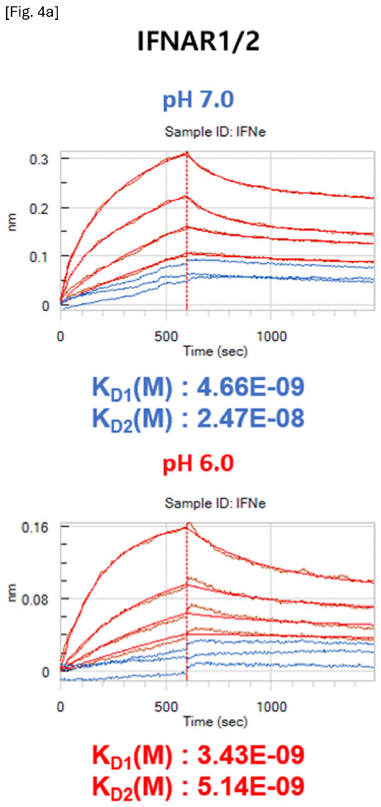

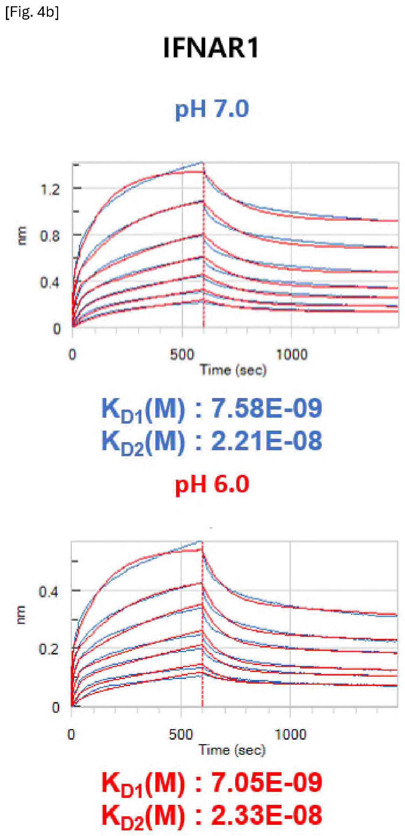

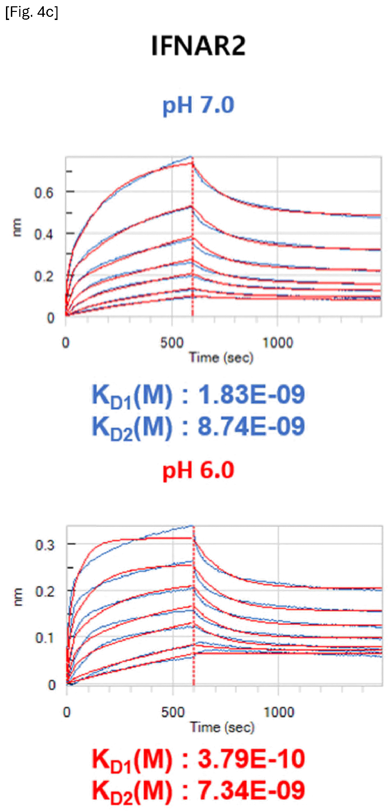

Fig. 4a to Fig. 4c illustrate the calculated results of binding kinetics and affinity of interferon epsilon to receptors IFNAR1/2, IFNAR1, and IFNAR2 under acidic conditions compared to neutral conditions.
Fig. 5a to Fig. 5c illustrate the results of comparing the expression levels of pSTAT-1 to confirm the degree of cellular signal transduction of interferon epsilon under acidic conditions compared to neutral conditions.
Fig. 6 illustrates an experimental schematic diagram for evaluating the side effects and efficacy of interferon epsilon in an in vivo mouse model.
Fig. 7a to Fig. 7g illustrate the blood analysis results of the *in vivo* mouse model experiment.
Fig. 8a to Fig. 8c illustrate the spleen analysis results of the *in vivo* mouse model experiment.
Fig. 9a to Fig. 9c illustrate the tumor analysis results of the *in vivo* mouse model experiment.

### [Detailed Description of the Invention]

The present invention will now be described in detail.

However, the following examples are merely illustrative of the present invention, and the scope of the present invention is not limited to the following examples.

### Example 1. Evaluation of receptor binding ability of type I interferons under neutral and acidic conditions

To evaluate the receptor binding ability of type I interferons, IFNAR1/2 fusion proteins were produced in Expi293F cells. Human IFNAR1-Fc and IFNAR2-Fc proteins, to which the knob-into-hole technology was applied, were inserted into the pOptiVec-TOPO plasmid (Invitrogen), and transfection was performed using the ExpiFectamine^{™} 293 Transfection Kit (Gibco #A14524). The transfected Expi293F cells were cultured for one week, after which the supernatant was collected and purified using a Protein A column on the AKTA avant (Cytiva) system.

The receptor binding ability of type I interferons under neutral and acidic conditions was evaluated using the ELISA method. Type I interferons used herein include Rebif (Merck), ABN101, ABN102 (Abion), IFN-α2b (BioLegend, #592704), IFN-ω (PBL, #11395-1), and IFN-ε (R&D Systems, #9667-ME-025/CF). A 96-well ELISA plate (Corning, #2592) was coated with interferon epsilon diluted to 100nM in PBS buffer, with 100ul applied per well, and incubated at 4°C for 16 hours. All buffers except the coating buffer were adjusted to pH 7.4 and pH 6.0 using 1N HCl. The coated plates were washed three times with washing buffer (0.05% PBS-T, pH 7.4, pH 6.0), followed by blocking with 300ul of blocking buffer (50mg/mL BSA in PBS, pH 7.4, pH 6.0) at room temperature for 1 hour. After blocking, the plates were washed three times with washing buffer, and IFNAR1/2 fusion proteins were serially diluted from 100nM at a 1/3 dilution ratio in reagent diluent (1mg/mL BSA in 0.05% PBS-T, pH 7.4, pH 6.0) and applied at 100ul per well, followed by incubation at room temperature for 1 hour. After washing three times with washing buffer, Anti-human IgG-HRP (Jackson lab, #109-035-003) was diluted 1:5000 and applied at 100ul per well, followed by incubation at room temperature for 1 hour. After washing three times with washing buffer, 100ul of TMB solution (Surmodics, #TMBW 1000-01) was applied, and the reaction was allowed to proceed at room temperature for 20 minutes. Absorbance was measured at 450nm after the reaction.

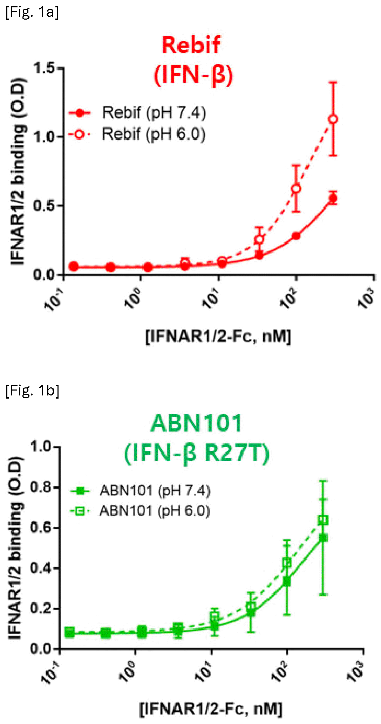

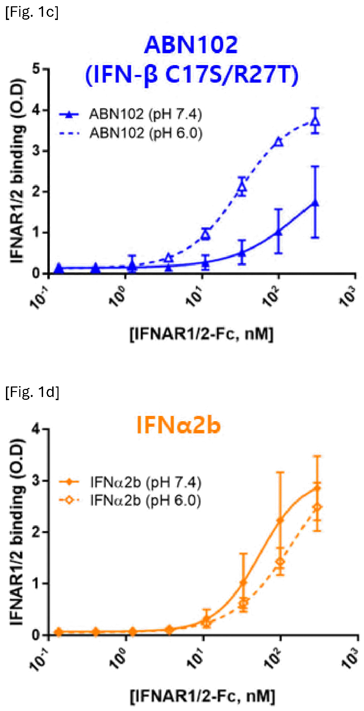

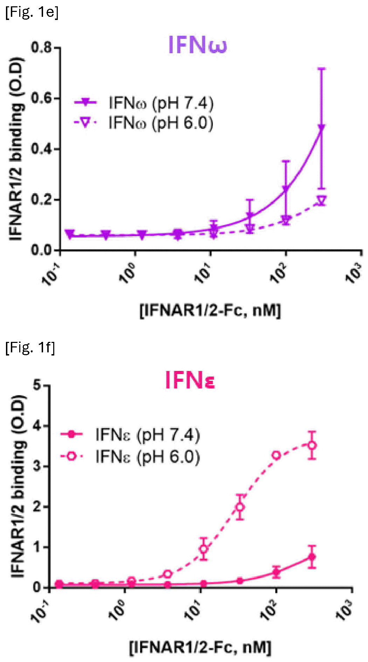

As a result of evaluating the receptor binding ability of the tested type I interferons according to pH, as shown in Figures 1a to 1f, Rebif, ABN101, ABN102, and IFN-ε exhibited superior receptor binding ability under acidic conditions compared to neutral conditions, with IFN-ε showing the most outstanding binding ability under acidic conditions.

### Example 2. Changes in binding ability of each type of type I interferons under acidic conditions compared to neutral conditions

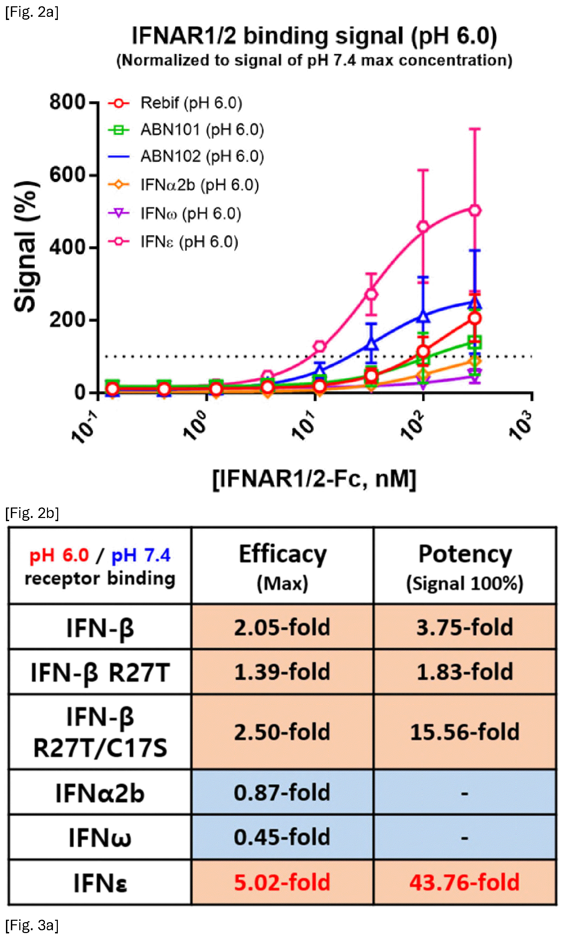

The binding ability of type I interferons evaluated using the ELISA method was standardized to neutral conditions and visualized (refer to Figures 2a and 2b). Rebif, ABN101, ABN102, and IFN-ω exhibited superior receptor binding ability under acidic conditions compared to neutral conditions. IFN-ε showed a 5.02-fold improvement in binding ability under acidic conditions compared to neutral conditions at the highest concentration and exhibited superior binding ability even when treated with 43.76 times lower receptor concentrations than under neutral conditions.

Through this, it was confirmed that IFN-ε has superior binding ability under acidic conditions compared to neutral conditions.

### Example 3. Changes in binding ability of interferon epsilon with IFNAR1 and IFNAR2 under acidic conditions compared to neutral conditions

To evaluate the receptor binding ability of type I interferons, human IFNAR1 and IFNAR2 fusion proteins were produced in Expi293F cells. Human IFNAR1-Fc and IFNAR2-Fc protein genes, to which the knob-into-hole technology was applied, were inserted into the pOptiVec-TOPO plasmid (Invitrogen), and transfection was performed using the ExpiFectamine^{™} 293 Transfection Kit (Gibco #A14524). The transfected Expi293F cells were cultured for one week, after which the supernatant was collected and purified using a Protein A column on the AKTA avant (Cytiva) system.

The receptor binding ability of type I interferons under neutral and acidic conditions was evaluated using the ELISA method. Type I interferons were produced by inserting human IFN-ε genes into the pOptiVec-TOPO plasmid (Invitrogen) and using the ExpiFectamine^{™} 293 Transfection Kit (Gibco, #A14524). A 96-well ELISA plate (Corning, #2592) was coated with interferon epsilon diluted to 100nM in PBS buffer, with 100ul applied per well, and incubated at 4°C for 16 hours. All buffers except the coating buffer were adjusted to pH 7.4 and pH 6.0 using 1N HCl. The coated plates were washed three times with washing buffer (0.05% PBS-T, pH 7.4, pH 6.0), followed by blocking with 300ul of blocking buffer (50mg/mL BSA in PBS, pH 7.4, pH 6.0) at room temperature for 1 hour. After blocking, the plates were washed three times with washing buffer, and IFNAR1/2 fusion proteins were serially diluted from 100nM at a 1/3 dilution ratio in reagent diluent (1mg/mL BSA in 0.05% PBS-T, pH 7.4, pH 6.0) and applied at 100ul per well, followed by incubation at room temperature for 1 hour. After washing three times with washing buffer, Anti-human IgG-HRP (Jackson lab, #109-035-003) was diluted 1:5000 and applied at 100ul per well, followed by incubation at room temperature for 1 hour. After washing three times with washing buffer, 100ul of TMB solution (Surmodics, #TMBW 1000-01) was applied, and the reaction was allowed to proceed at room temperature for 20 minutes. Absorbance was measured at 450nm after the reaction.

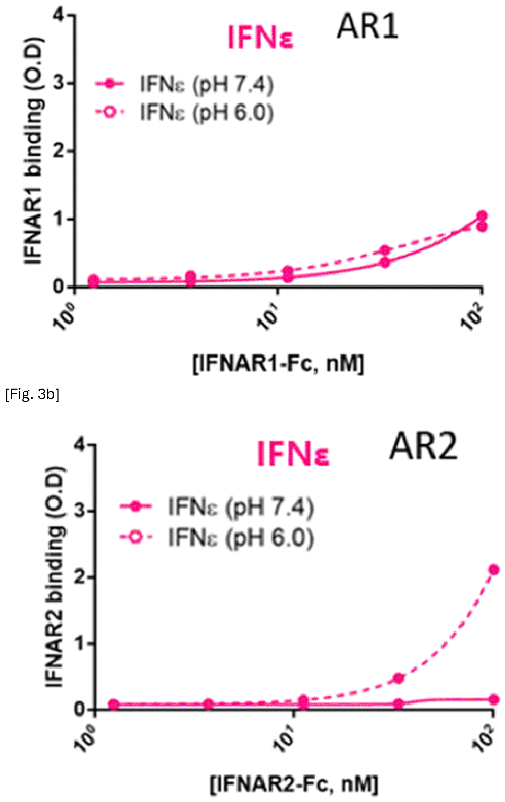

As a result of evaluating the binding ability of interferon epsilon with IFNAR1 and IFNAR2 according to pH, as shown in Figures 3a and 3b, it was confirmed that interferon epsilon exhibited stronger receptor binding under acidic conditions in an acid-dependent manner via IFNAR2.

### Example 4. Receptor binding kinetics and affinity of interferon epsilon under acidic conditions compared to neutral conditions

To confirm the receptor binding kinetics of interferon epsilon under neutral and acidic conditions, analysis was performed using the Octet R8 (Sartorius) instrument. hIFNAR1/2, hIFNAR1, and hIFNAR2 were each coated at 20nM on an AHC biosensor (Sartorius) using 1x kinetics buffer. The 1x kinetics buffer was adjusted with 1N HCl according to neutral and acidic analysis conditions.

Interferon epsilon was diluted from 200nM in 1/2-fold steps and allowed to bind to the receptors coated on the AHC biosensor, and kinetics were calculated through the dissociation process using the Octet R8 instrument.

As a result, similar to previous experimental results, it was confirmed that interferon epsilon had a lower KD value under acidic conditions compared to neutral conditions and exhibited stronger binding under acidic conditions with AR2 than with AR1.

### Example 5. Confirmation of signaling activation by interferon epsilon in Daudi cells and human CD3 T cells under acidic conditions compared to neutral conditions

To confirm the difference in cellular signaling activation through receptor binding of interferon epsilon under neutral and acidic environments, Daudi cells, which are human B lymphoblast cells, and CD3 T cells isolated from healthy donor human peripheral blood mononuclear cells (PBMCs) were used. CD3 T cells from human PBMCs were isolated using the human CD3 T cell isolation kit (Miltenyl Biotec).

The cell culture medium was adjusted to pH 7.4 and pH 6.5 using 1N HCl in RPMI1640, followed by the addition of 20mM HEPES. Daudi cells and CD3 T cells were seeded at 2x10⁵ cells per well in a 96-well tissue culture plate, and interferon epsilon was diluted from 100nM in 1/10-fold steps in pH 7.4 and pH 6.5 media and treated for 24 hours. After 24 hours, the cells were collected, separated into single cells using cell separation buffer, and lysed with RIPA buffer (BIOSESANG) containing protease and phosphatase inhibitors at 200rpm, 4°C for 1 hour. The lysate was centrifuged at 15,000rpm, 4°C for 15 minutes, and only the supernatant was collected. The dissolved proteins were quantified using a BCA assay (ThermoFisher Scientific). DTT-containing 5X sample loading buffer (BIOSESANG) was added to the quantified proteins, and the mixture was boiled for 10 minutes to reduce all proteins. Equal amounts of protein were loaded onto SDS-PAGE, separated by size, transferred to a PVDF membrane, and blocked with 5% BSA in TBS-T at 4°C for 1 hour. Anti-human pSTAT-1 (Cell Signaling Technology) was used as the primary antibody at a 1:1000 ratio and incubated overnight on the PVDF membrane. After washing three times with 0.05% TBS-T, the secondary antibody, anti-rabbit Fc HRP antibody (Invitrogen), was applied at a 1:5000 ratio and incubated for 30 minutes. After washing three times with 0.05% TBS-T, ECL solution (Bio-Rad) was added, and detection was performed using Chemidoc.

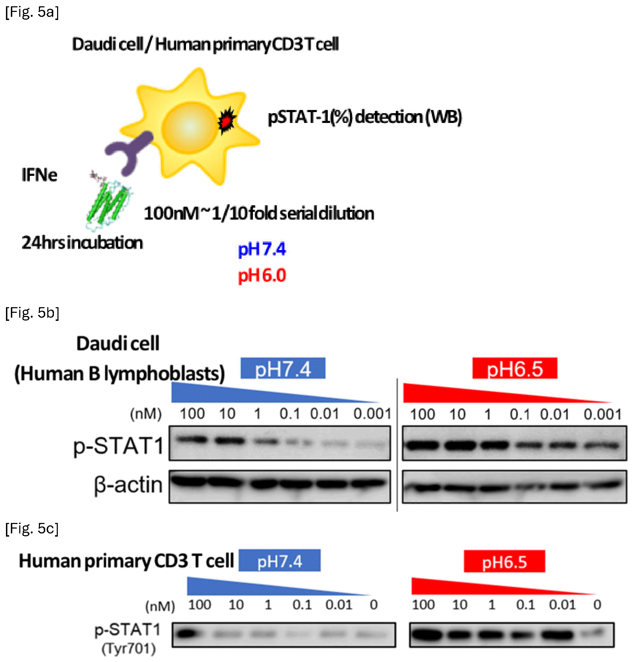

As a result of evaluating the cellular signaling activation of the tested type I interferons according to pH, as shown in Figures 5a to 5c, it was confirmed that interferon epsilon activated pSTAT-1 signaling in Daudi cells and human CD3 T cells under acidic conditions at concentrations 1000 times lower than those under neutral conditions.

### Example 6. Evaluation of side effects and efficacy of interferon epsilon in an in vivo mouse model

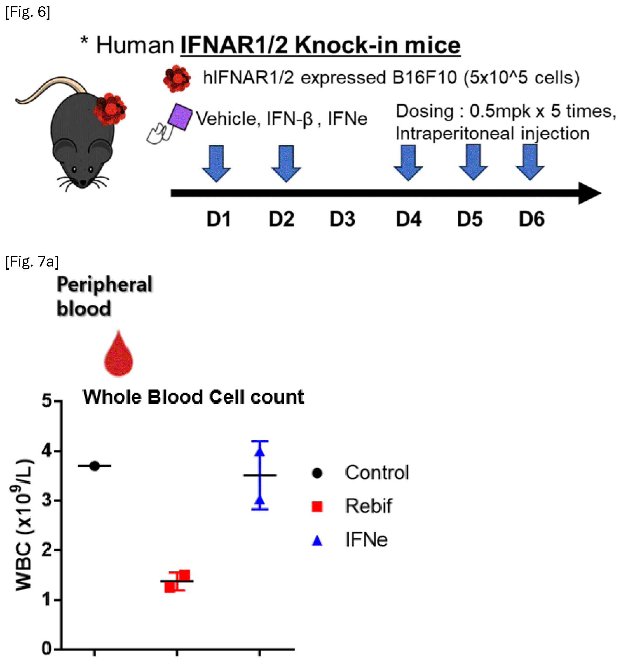

To evaluate the side effects and efficacy of interferon epsilon in an *in vivo* mouse model, human hIFNAR1/2 knock-in (hIFNAR1/2 KI) mice were used. A tumor model was induced by subcutaneously injecting 5x10⁵ cells of the B16F10 cell line expressing human hIFNAR1/2 into hIFNAR1/2 KI mice. When the tumor size reached 100-150mm³, the mice were divided into control, IFN-β, and IFN-ε treatment groups, and 0.5mg/kg doses were administered intraperitoneally for 5 days. The experimental schematic is shown in Figure 6A. One hour after the final administration, blood, spleen, and tumors were collected and analyzed using a flow cytometer.

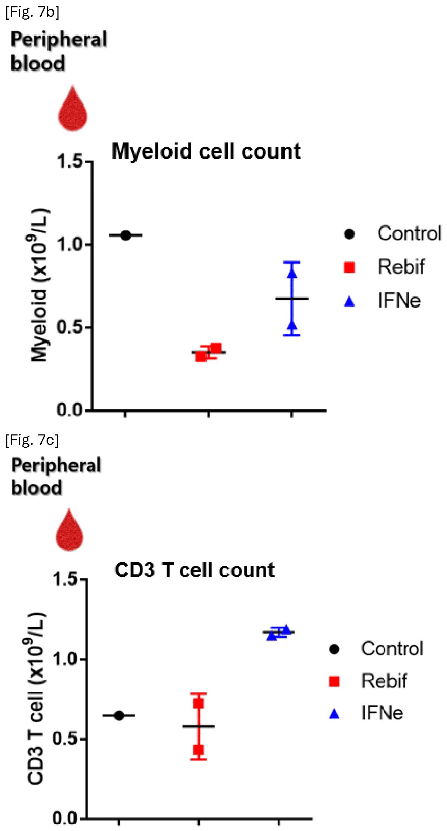

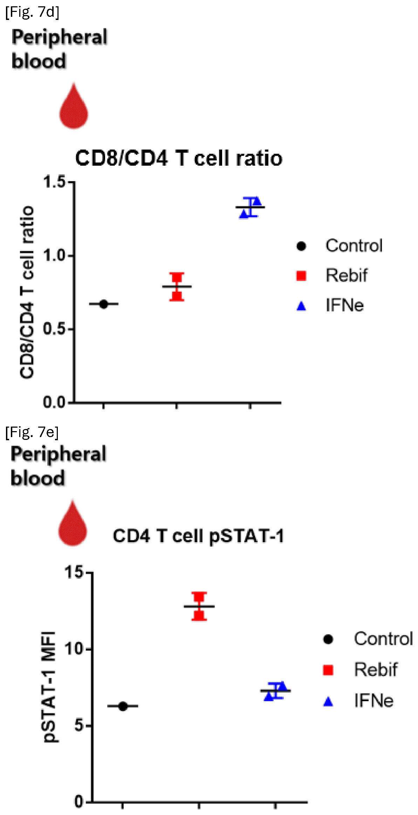

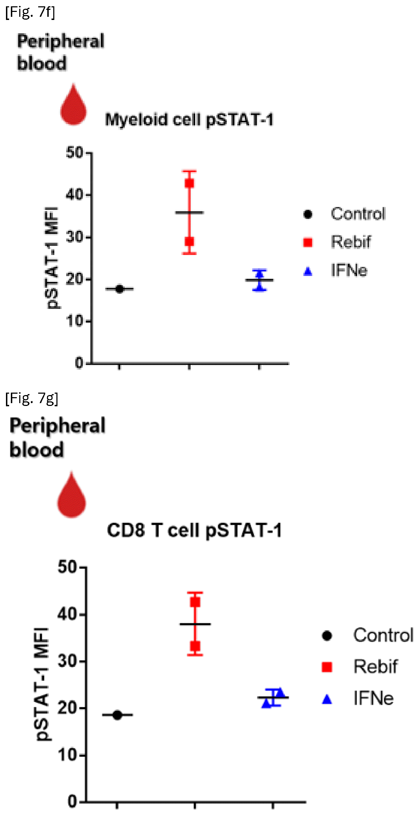

As shown in Figures 7a to 7g, blood analysis results confirmed that interferon epsilon, which exhibits increased activity under acidic conditions, caused less reduction in white blood cell count, a marker of neutropenia side effects, compared to interferon beta. It also increased CD8 T cells through T cell activation while reducing immunosuppressive myeloid cells. Furthermore, it did not cause excessive activation of pSTAT-1, which is involved in downstream signaling in the blood.

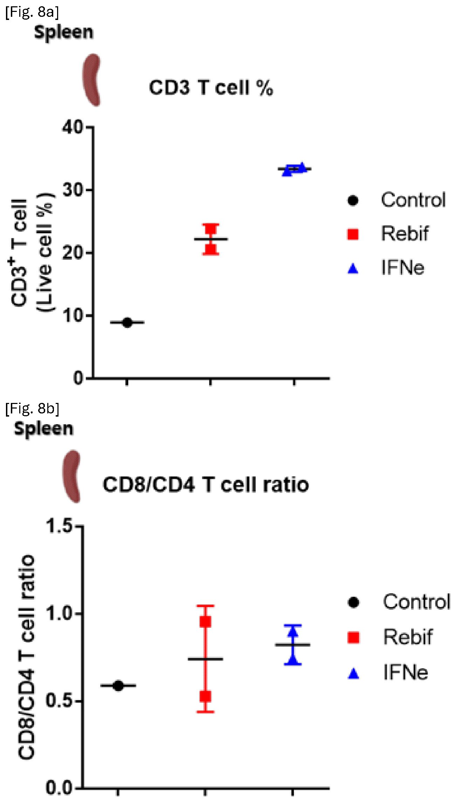

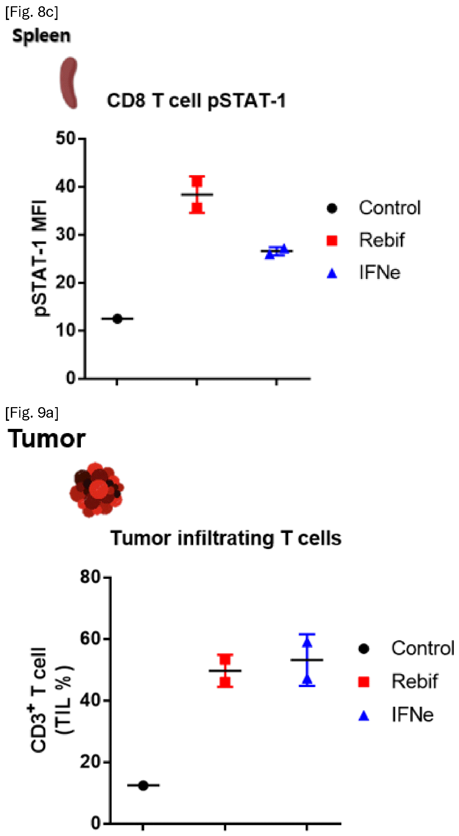

As shown in Figures 8a to 8c, spleen analysis results confirmed that interferon epsilon increased the proportion of T cells more than interferon beta and did not cause excessive activation of pSTAT-1.

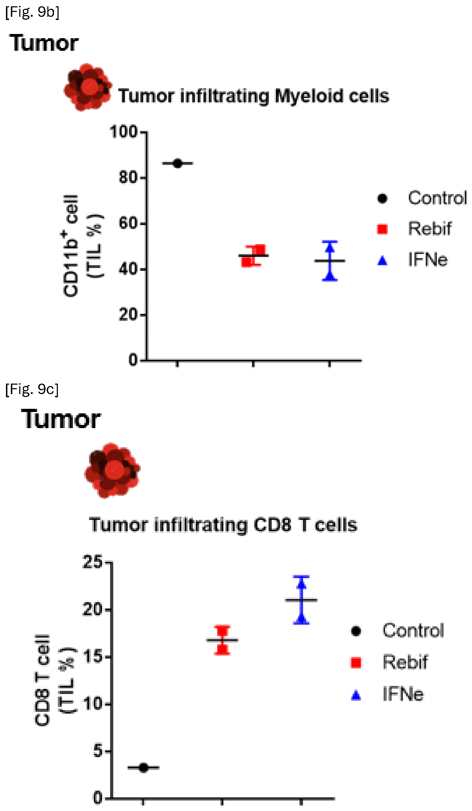

As shown in Figures 9a to 9c, tumor analysis results confirmed that interferon epsilon increased T cell infiltration into the tumor through antitumor immune activation similar to interferon beta and reduced immunosuppressive myeloid cells.

In the *in vivo* mouse model, interferon epsilon exhibited less nonspecific immune activation in the blood and spleen compared to interferon beta, while showing similar levels of immune activation within the tumor. Based on this, it was confirmed that interferon epsilon, with increased activity under acidic conditions, has superior antitumor efficacy compared to other interferon subtypes.

### [Industrial Applicability]

As described above, interferon epsilon according to the present invention is characterized by significantly enhanced activity in a mildly acidic environment compared to a neutral environment. Therefore, it has the advantage of being capable of systemic administration without additional processes such as targeting or masking to control systemic toxicity, and thus can be usefully utilized in the treatment of related diseases.

## Claims

1. A pharmaceutical composition for preventing or treating a disease comprising interferon epsilon as an active ingredient, wherein the interferon epsilon is activated in tissue with decreased pH due to a pathological state of the disease.

2. The pharmaceutical composition of claim 1, wherein the composition is for systemic administration.

3. The composition of claim 1, wherein the disease is cancer or an infectious disease.

4. The composition of claim 1, wherein the pH of the tissue with decreased pH is 5.0 to 6.8.

5. The composition of claim 1, wherein the activity of the interferon epsilon is increased 2-fold to 1000-fold at pH 6.0 compared to pH 7.4.

6. The composition of claim 2, wherein the systemic administration is intravenous, intramuscular, intraarterial, intramedullary, intrathecal, intracardiac, transdermal, subcutaneous, intraperitoneal, intranasal, enteral, topical, sublingual, intrarectal, or intratumoral administration.

7. The composition of claim 3, wherein the cancer is at least one selected from the group consisting of ovarian cancer, papilloma, laryngeal papilloma, human papilloma, bladder cancer, cervical cancer, hairy cell leukemia, chronic myelogenous leukemia, multiple myeloma, non-Hodgkin's lymphoma, skin cancer such as melanoma, Kaposi's sarcoma, kidney cancer, lung cancer, brain tumor such as glioma, breast cancer, colorectal cancer, liver cancer, hepatocellular carcinoma, gastric cancer, bronchial cancer, nasopharyngeal cancer, laryngeal cancer, pancreatic cancer, colon cancer, pancreatic cancer, prostate cancer, bone cancer, thyroid cancer, parathyroid cancer, esophageal cancer, bile duct cancer, testicular cancer, rectal cancer, head and neck cancer, cervical cancer, ureteral cancer, neuroblastoma, fibrosarcoma, rhabdomyosarcoma, astrocytoma, glioblastoma, neuroblastoma, and malignant mesothelioma.

8. The composition of claim 3, wherein the infectious disease is at least one selected from the group consisting of AIDS caused by HIV, hepatitis B and C virus infection, influenza virus infection, respiratory syncytial virus infection, herpes virus infection, human papilloma virus infection, and corona virus infection.

9. A fusion protein comprising interferon epsilon and an antibody or fragment thereof directly or indirectly bound to the interferon epsilon.

10. The fusion protein of claim 9, wherein the fusion protein is **characterized by** an interferon epsilon linked to an antibody or fragment thereof via a linker.

11. A pharmaceutical composition for preventing or treating a disease comprising the fusion protein according to claim 9 or 10, wherein the fusion protein is activated in tissue with decreased pH due to a pathological state of the disease.

12. The pharmaceutical composition of claim 11, wherein the composition is for systemic administration.

13. The composition of claim 11, wherein the disease is cancer or an infectious disease.

14. The composition of claim 11, wherein the pH of the tissue with decreased pH is 5.0 to 6.8.

15. The composition of claim 11, wherein the activity of the interferon epsilon is increased 2-fold to 1000-fold at pH 6.0 compared to pH 7.4.

16. The composition of claim 12, wherein the systemic administration is intravenous, intramuscular, intraarterial, intramedullary, intrathecal, intracardiac, transdermal, subcutaneous, intraperitoneal, intranasal, enteral, topical, sublingual, intrarectal, or intratumoral administration.

17. The composition of claim 13, wherein the cancer is at least one selected from the group consisting of ovarian cancer, papilloma, laryngeal papilloma, human papilloma, bladder cancer, cervical cancer, hairy cell leukemia, chronic myelogenous leukemia, multiple myeloma, non-Hodgkin's lymphoma, skin cancer such as melanoma, Kaposi's sarcoma, kidney cancer, lung cancer, brain tumor such as glioma, breast cancer, colorectal cancer, liver cancer, hepatocellular carcinoma, gastric cancer, bronchial cancer, nasopharyngeal cancer, laryngeal cancer, pancreatic cancer, colon cancer, pancreatic cancer, prostate cancer, bone cancer, thyroid cancer, parathyroid cancer, esophageal cancer, bile duct cancer, testicular cancer, rectal cancer, head and neck cancer, cervical cancer, ureteral cancer, neuroblastoma, fibrosarcoma, rhabdomyosarcoma, astrocytoma, glioblastoma, neuroblastoma, and malignant mesothelioma.

18. The composition of claim 13, wherein the infectious disease is at least one selected from the group consisting of AIDS caused by HIV, hepatitis B and C virus infection, influenza virus infection, respiratory syncytial virus infection, herpes virus infection, human papilloma virus infection, and corona virus infection.
